# EUROPÄISCHE PATENTSCHRIFT

(11) **EP 3 248 646 B1**
(45) Veröffentlichungstag und Bekanntmachung des Hinweises auf die Patenterteilung: **17.02.2021**
(21) Anmeldenummer: 16171312.8
(22) Anmeldetag: 25.05.2016
(51) Int. Cl.: A61M 31/00, B65D 75/58, B65D 1/09, B65D 35/08

(54) **ZWEITEILIGES KUNSTSTOFFROHLING-SET**
TWO-PART PLASTIC BLANK SET
ENSEMBLE D'EBAUCHES EN PLASTIQUE EN DEUX PARTIES

(43) Veröffentlichungstag der Anmeldung: 29.11.2017
(73) Patentinhaber: Mattern, Claudia, 6376 Emmetten (CH)
(72) Erfinder: Mattern, Claudia, 6376 Emmetten (CH)
(74) Vertreter: Scholz, Volker

(56) Entgegenhaltungen:
- EP-B1- 2 626 304
- US-A- 5 215 221
- US-A1- 2012 024 905
- US-A1- 2012 223 075
- US-A1- 2012 312 709

## Beschreibung

Die vorliegende Erfindung betrifft ein zweiteiliges Kunststoffrohling-Set, bevorzugt zur Herstellung eines Wirkstoffapplikators zur Aufbewahung einer Einheitsdosis eines Wirkstoffpräparats und zur Verabreichung dieser Einheitsdosis in die menschliche Nase; sowie einen daraus herstellbaren Wirkstoffapplikator.

Bei bestimmten Wirkstoffpräparaten für den menschlichen Körper kommt es auf eine exakte Dosierung an. Um eine solche exakte Dosierung durch den Benutzer zu gewährleisten, werden Dosiereinheiten bereitgestellt, die die vorgesehene Menge des Wirkstoffpräparates enthalten, und die bei der Anwendung genau diese vorgesehene Menge dem menschlichen Körper zuführen. Darüber hinaus sind auch Wirkstoffapplikatoren bekannt, die für die einmalige Benutzung vorgesehen sind und dabei eine Doppelfunktion ausüben: Zum einen enthalten sie eine fest vorgegebene Dosiermenge des Wirkstoffpräparates. Zum anderen dienen sie nicht nur der Bereitstellung, sondern auch der Zufuhr des Wirkstoffpräparates in der gewünschten Menge an die gewünschte Stelle. Derartige Wirkstoffapplikatoren, die ein kleines Volumen in der Größenordnung von einem Kubikzentimeter oder weniger haben können, haben im allgemeinen die Form eines kleinen Fläschchens und werden zur Abgabe des Behälterinhalts seitlich zusammengedrückt, um den Behälterinhalt auszupressen. Nach erfolgter Anwendung wird die entleerte Einheit entsorgt. Ein solcher Wirkstoffapplikator für die Bereitstellung und Verabreichung einer Einheitsdosis in die Nase ist aus dem eingetragenen Gemeinschaftsgeschmacksmuster 001790908-0001 oder der DE 10 2010 033 015 A1 bekannt.

Der genannte Wirkstoffapplikator weist ein Unterteil und ein Deckelteil auf, zwischen denen ein Aufnahmeraum für ein Wirkstoffpräparat ausgebildet ist. Das Unterteil und das Deckelteil weisen je einen umlaufenden Siegelrand zur dichtenden Verbindung des Deckelteils mit dem Unterteil auf. Das Unterteil ist mit einer Applikationsröhre versehen, die in den Aufnahmeraum mittels einer inneren Mündung mündet. Bezogen auf die Ebene der gefügten Siegelränder ragt die für die Einführung in die Nase vorgesehene Applikationsröhre in einem flachen Winkel ausgehend vom Aufnahmeraum über die Siegelränder hervor und deckt dadurch einen Stützflächenabschnitt des Unterteil-Siegelrandes ab. Unterteil und Deckelteil sind über ein Filmscharnier einteilig ausgebildet.

Ein einteiliger Kunststoffrohling zur Bildung eines solchen Wirkstoffapplikators kann als Spritzgussteil kostengünstig unter den Bedingungen einer Großserienfertigung hergestellt werden. Das Befüllen und Verschließen dieses Kunststoffrohlings gestaltet sich jedoch schwierig wegen der geometrischen Anordnung der Applikationsröhre. Für die Befüllung ist eine lagegenaue Positionierung des noch offenen Rohlings erforderlich, bei der die Applikationsröhre im Wege ist. Für den anschließenden Verschließvorgang, bei dem das Deckelteil auf das Unterteil mit der Applikationsröhre aufgesiegelt wird, muss am Siegelrand des Unterteils ein Gegendruck aufgebracht werden, der sich über die gesamte Siegelfläche erstreckt. Nur so kann eine ringsum wirkende, hermetisch verschlossene Versiegelung sichergestellt werden. Im Bereich der Applikationsröhre ist aber der Siegelrand durch diese abgedeckt, so dass hier der Zugriff auf den Siegelrand zum Aufbringen des Gegendrucks verwehrt ist.

Es hat sich bei der technischen Umsetzung gezeigt, dass eine einstückige Ausbildung von Deckelteil und Unterteil nachteilig ist, da eine industrielle Fertigung und Befüllung eines aus einem einteiligen Werkstück gebildeten Applikators nicht durchführbar ist, insbesondere Schwierigkeiten bei der korrekten Einlegung des einstückigen Kunststoffrohlings in eine Füll- und Verschließeinrichtung bestehen. Insbesondere kann das einteilige Werkstück nicht vollautomatisch befüllt und verschlossen werden

Siehe auch EP2626304 A1, US2012312709 A1, US5215221 A und US2012024905 A1.

Darüber hinaus hat sich gezeigt, dass ein Ansatzelement zwischen Applikationsröhre und Verschluss während des Transports häufig angeknickt wird, wodurch Undichtigkeiten entstehen können.

Es ist daher Aufgabe der vorliegenden Erfindung, ein Kunststoffrohling-Set zur Herstellung eines Wirkstoffapplikators, bevorzugt zur Aufbewahrung einer Einheitsdosis eines Wirkstoffpräparats und zur Verabreichung dieser Einheitsdosis in die menschliche Nase, bereitzustellen, das die Nachteile des Stands der Technik überwindet, insbesondere ein einfaches und schnelles Füllen und Verschließen in einer entsprechenden Füll- und Verschießeinrichtung erlaubt sowie Undichtigkeiten beim Transport eines daraus hergestellten Wirkstoffapplikators vermeidet.

Diese Aufgabe wird gelöst durch ein zweiteiliges Kunststoffrohling-Set, bevorzugt zur Herstellung eines Wirkstoffapplikators zur Aufbewahrung einer Einheitsdosis eines Wirkstoffpräparats und zur Verabreichung dieser Einheitsdosis in die menschliche Nase, wobei das Kunststoffrohling-Set ein Unterteil und ein vom Unterteil getrenntes Deckelteil umfasst, wobei das Unterteil eine von einem Siegelrand umschlossene Aufnahmeraumausformung aufweist und sich von der Aufnahmeraumausformung eine einteilig angeformte Applikationsröhre erstreckt, die an ihrem freien Ende mit einem einteilig über ein Ansatzelement angeformten Verschluss verschlossen ist, wobei das Ansatzelement eine Wandstärke von 0,1-0,5 mm, bevorzugt 0,2-0,3 mm aufweist, und wobei das Deckelteil eine Größe aufweist, um bei Auflegen auf das Unterteil die Authahmeraumausformung desselben vollständig abzudecken und einen Siegelrand aufweist, der dichtend mit dem Siegelrand des Unterteils verbindbar ist.

Dabei ist vorgesehen, dass zumindest am Deckelteil oder am Unterteil ein sich vom jeweiligen Siegelrand, bevorzugt in derselben Ebene, erstreckendes Ansatzplättchen angeformt ist.

Das sich von einem Siegelrand oder beiden Siegelrändern erstreckende Ansatzplättchen ist insbesondere hilfreich, wenn Produktinformationen auf dem Deckelteil oder dem Unterteil angebracht werden sollen. Dies kann beispielsweise durch Anbringung eines Etiketts am Ansatzplättchen oder durch Bedruckung des Ansatzplättchens erfolgen. Bei Verwendung eines Etiketts wird verhindert, dass Klebstoff in die Aufnahmeraumausformung gelangen kann. Weisen sowohl der Siegelrand des Unterteils als auch der Siegelrand des Oberteils daran angesetzte und beim fertigen Wirkstoffapplikator sich gegenüberliegende Ansatzplättchen auf, können diese ebenfalls zusammen versiegelt werden, was die Dichtigkeit und Stabilität des hergestellten Wirkstoffapplikators noch verbessert.

Desweiteren ist vorgesehen, dass zumindest der Siegelrand des Deckelteils oder der Siegelrand des Unterteils mit einen Energierichtungsgeber auf der dem Unterteil bzw. dem Oberteil gegenüberliegenden Seite ausgeformt ist.

Durch die Bereitstellung von Energierichtungsgebern an den Siegelrändern des Deckelteils und/oder Unterteils können Ultraschallschweißergebnisse prozesssicher gestaltet werden. Energierichtungsgeber sind auf dem Gebiet der Ultraschallschweißtechnik bekannt und haben insbesondere die Aufgabe, die Plastifizierung der Fügeflächen durch Energiekonzentration schnell einzuleiten. Bei Einsatz eines Energierichtungsgebers kommt es zu einer Nahtbildung an der Schweißstelle, wodurch Festigkeit und Einheitlichkeit des Schweißergebnisses verbessert werden. Diesbezüglich ist zumindest ein Siegelrand auf der dem anderen Siegelrand gegenüberliegenden Fläche bevorzugt mit kleinen Rillen versehen.

Ferner ist bevorzugt vorgesehen, dass die Aufnahmeraumausformung kugelkappenförmig oder kugelabschnittförmig ist. Die Aufnahmeraumausformung kann jedoch auch, beispielsweise, einen ovalen Grundriss haben, wobei der umlaufende Siegelrand sowie der Siegelrand des Deckelteils dann ebenfalls passend ovalförmig sind.

Schließlich ist vorgesehen, dass das Deckelteil im wesentlichen eben ausgebildet ist.

Erfindungsgemäß ist auch ein Wirkstoffapplikator, der durch Verschweißen, vorzugsweise Ultraschall-Verschweißen, des Unterteils mit dem Deckelteil des zweiteiligen erfindungsgemäßen Kunststoffrohling-Sets gemäß Anspruch 1 über die jeweiligen Siegelränder herstellbar ist und mit einem Wirkstoff befüllt ist

Die an der Aufnahmeraumausformung angformte Applikationsröhre ist zur Aufnahmeraumausformung hin offen. Das freie Ende der Applikationsröhre ist jedoch durch einen Verschluss, vorzugsweise einen abreißbaren Stopfen oder Pin verschlossen. Der Verschluss ist mit der Applikationsröhre über ein Ansatzelement, vorzugsweise eine Abreißnaht, verbunden. Zur Abgabe des Inhalts des Wirkstoffapplikators wird der Verschluss, z.B. durch Drehen um seine Längsachse, von der Applikationsröhre entfernt. Vorzugsweise befindet sich das Ansatzelement im Inneren der Applikationsröhre, so dass beim Gebrauch kein verbleibender Grat von der Applikationsröhre vorsteht, so dass es keine Verletzung hervorrufen kann, wenn es beispielsweise in die Nase eines Benutzers eingeführt wird.

Der erfindungsgemäß hergestellte Wirkstoffapplikator zeichnet sich insbesondere durch einen durch das Deckelteil und das Unterteil gebildeten Aufnahmeraum aus, der eine nach außen gewölbte Wand und eine gegenüberliegende Wand aufweist, die mit einem umlaufenden Steg versehen ist, der in den Hohlraum der gewölbten Wand an dessen Rand eingreift, wobei die gewölbte Wand so geformt ist, dass sie an den Steg und den dazwischenliegenden Bereich der gegenüberliegenden Wand glatt anpressbar ist.

Dabei liegt im zusammengepressten Zustand die gewölbte Wand im wesentlichen ohne verbleibenden Zwischenraum an den Steg und der gegenüberliegenden Wand an, so dass praktisch der gesamte Inhalt aus dem Aufnahmeraum herausgedrückt wird, ohne dass eine nennenswerte Restmenge zurückbleiben kann, da sich die gewölbte Wand glatt, ohne Faltenbildung, um den Steg herum an die gegenüberliegende Wand anlegt. Der Steg, der vorzugsweise querschnittlich eine im wesentlichen konische Form hat, mit gerundeter Spitze und leicht gerundeten Flanken, füllt dabei den Raum aus, der sich beim Zusammendrücken einer gewölbten Wand an deren äußeren Rand zwangsläufig ausbildet. Da dieser Raum durch den Steg ausgefüllt ist, kann dort keine Restmenge des Behälterinhalts zurückbleiben.

Als Material für das Deckelteil und das Unterteil sind beispielsweise Polyolefine, wie Polypropylen oder Polyethylen, geeignet, ohne dass die Erfindung hierauf beschränkt ist.

Es wurde erfindungsgemäß überraschend gefunden, dass durch Bereitstellung eines zweiteiligen Kunststoffrohling-Sets produktionstechnische Schwierigkeiten beim Füllen und Verschließen eines daraus hergestellten Wirkstoffapplikators überwunden werden können. Erst durch die Bereitstellung dieses zweiteiligen Kunststoffrohling-Sets ist eine industrielle Produktion von Wirkstoffapplikatoren möglich, beispielsweise mit einer Herstellungsgeschwindigkeit von mindestens 200 Wirkstoffapplikatoren pro Minute. Das ist in etwa das 10-Fache dessen, was bei einer Befüllung eines einteiligen Applikators bisher möglich war. Die vollautomatische Befüllung des erfindungsgemäßen Wirkstoffapplikators erlaubt eine Dosis-genauere Befüllung und ein 100%iges Ultraschallverschweißen.

Darüber hinaus wurde auch überraschend festgestellt, dass durch geeignete Einstellung der Wandstärke des Ansatzelements zwischen Applikationsröhre und Verschluss die Transportsicherheit der hergestellten Wirkstoffapplikatoren verbessert werden kann. Insbesondere wird ein Anknicken des Ansatzelements verhindert, wodurch Undichtigkeiten vermieden werden.

Weitere Einzelheiten der Erfindung ergeben sich aus der nachfolgenden Beschreibung sowie anhand der beiliegenden Zeichnungen. Dabei zeigt
Fig. 1 in perspektivischer Ansicht ein zweiteiliges Kunststoffrohling-Set aus Unterteil und Oberteil gemäß der Erfindung; und
Fig. 2 einen aus Deckelteil und Unterteil hergestellten Wirkstoffapplikator gemäß der Erfindung in perspektivischer Ansicht.

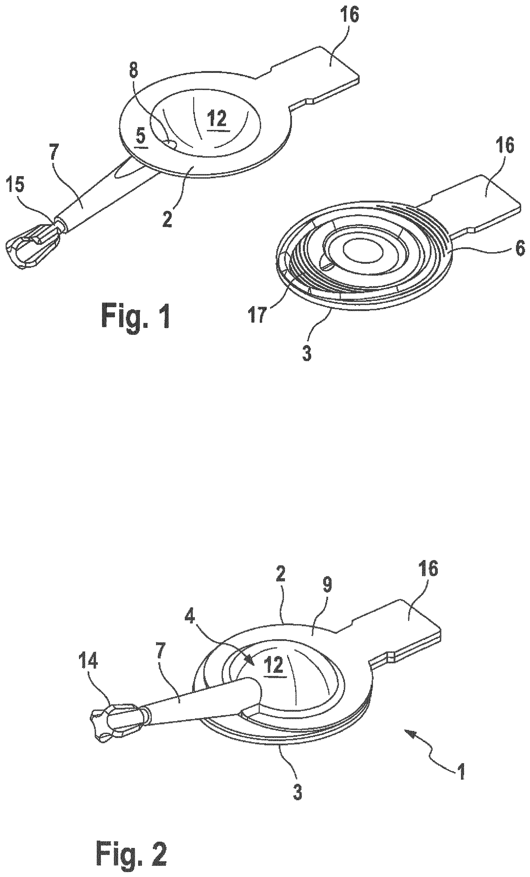

Fig. 1 zeigt in einer perspektivischen Ansicht ein zweiteiliges Kunststoffrohling-Set zur Herstellung eines Wirkstoffapplikators. Das Kunststoffrohling-Set umfasst ein Unterteil 2 sowie ein Deckelteil 3. Das Unterteil 2 weist eine von einem Siegelrand 5 umschlossene Aufnahmeraumausformung 12 auf, die im wesentlichen kugelkappenförmig ausgebildet ist. Ausgehend von der Aufnahmeraumausformung 12 erstreckt sich eine einteilig angeformte Applikationsröhre 7, die an ihrem freien Ende mit einem ebenfalls einteilig über ein Ansatzelement 15 angeformten Verschluss 14 verschlossen ist. Das Deckelteil 3 weist eine Größe auf, um die Aufnahmeraumausformung 12 des Unterteils 2 vollständig abdecken zu können. Das Deckelteil 3 weist ferner einen umlaufenden Siegelrand 6 auf, der dichtend mit dem Siegelrand 5 des Unterteils 2 verbindbar ist. Das Deckelteil 3 ist im wesentlichen eben ausgebildet und weist einen Siegelrand 6 mit einem Energierichtungsgeber in Form von kleinen Rillen 17 auf.

In der Aufnahmeraumausformung 12 ist eine Mündung 8 ausgebildet, mittels derer die Applikationsröhre 7 in die Aufnahmeraumausformung 12 mündet.

Sowohl das Unterteil 2 als auch das Deckelteil 3 weisen Ansatzplättchen 16 auf, die sich vom jeweiligen Siegelrand in derselben Ebene erstrecken. Diese Ansatzplättchen können auf deren dem anderen Teil abgewandten Seiten bedruckt oder mit einem Klebeetikett versehen werden. Zur Herstellung eines erfindungsgemäßen Wirkstoffapplikators 1, wie er in Fig. 2 gezeigt ist, aus dem erfindungsgemäßen zweiteiligen Kunststoffrohlung-Set wird das Unterteil 2 in einer geeigneten Füll- und Verschließeinrichtung mit einem Wirkstoff befüllt und anschließend mit dem Deckelteil 3 versiegelt.

Dazu kann das Unterteil in eine Füll- und Verschließeinrichtung eingelegt werden, wie sie beispielsweise aus der EP 2 626 304 B1 bekannt ist. Im Gegensatz zu dem dort beschriebenen Verfahren unter Verwendung eines einteiligen Rohlings wird gemäß der vorliegenden Erfindung zunächst das Unterteil 2 eingelegt und befüllt und nach Befüllung des Unterteils 2 das Deckelteil 3 so auf das Unterteil 2 aufgelegt, dass sein Siegelrand 6 auf dem um die Aufnahmeraumausformung 12 umlaufenden Siegelrand 5 des Unterteils 2 zu liegen kommt. Das Verschweißen, vorzugsweise Ultraschallverschweißen, erfolgt dann nach im Stand der Technik bekannten routinemäßigen Verfahren.

Ein so hergestellter Wirkstoffapplikator 1 ist in Fig. 2 gezeigt. Unterteil 2 und Oberteil 3 bilden zusammen einen Aufnahmeraum 4 auf, in dem ein Wirkstoff aufgenommen ist.

Für die Anwendung wird zunächst der Verschluss 14 über das Ansatzelement 15 abgebrochen bzw. abgedreht und entfernt. Anschließend wird die Applikationsröhre 7 in ein Nasenloch eingeführt, wobei dann durch Finger- bzw. Daumendruck auf die Aufnahmeraumausformung 12 das im Aufnahmeraum 4 vorgehaltene Wirkstoffpräparat durch die Applikationsröhre 7 in die Nase gedrückt wird. Das Ansatzelement 15 weist eine Wandstärke im Bereich von 0,1-0,5 mm, bevorzugt 0,2-0,3 mm, auf.

Die in der vorstehenden Beschreibung und den Ansprüchen offenbarten Merkmale der Erfindung können für die Verwirklichung der Erfindung gemäß Anspruch 1 in ihren unterschiedlichen Ausführungsformen wesentlich sein.

## Patentansprüche

1. Zweiteiliges Kunststoffrohling-Set, bevorzugt zur Herstellung eines Wirkstoffapplikators (1) zur Aufbewahrung einer Einheitsdosis eines Wirkstoffpräparats und zur Verabreichung dieser Einheitsdosis in die menschliche Nase, wobei das Kunststoffrohling-Set ein Unterteil (2) und ein vom Unterteil (2) getrenntes Deckelteil (3) umfasst, wobei das Unterteil (2) eine von einem Siegelrand (5) umschlossene Aufnahmeraumausformung (12) aufweist und sich von der Aufnahmeraumausformung (12) eine einteilig angeformte Applikationsröhre (7) erstreckt, die an ihrem freien Ende mit einem einteilig über ein Ansatzelement (15) angeformten Verschluss (10) verschlossen ist, wobei das Ansatzelement (15) eine Wandstärke von 0,1-0,5 mm aufweist, und wobei das Deckelteil (3) eine Größe aufweist, um bei Auflegen auf das Unterteil (2) die Aufnahmeraumausformung (12) desselben vollständig abzudecken und einen Siegelrand (6) aufweist, der dichtend mit dem Siegelrand (5) des Unterteils (2) verbindbar ist,
wobei zumindest am Deckelteil (3) oder am Unterteil (2) ein sich vom jeweiligen Siegelrand (5, 6), erstreckendes Ansatzplättchen (16) angeformt ist, und
wobei zumindest der Siegelrand (6) des Deckelteils (3) oder der Siegelrand (5) des Unterteils (2) mit einem Energierichtungsgeber (17) in Form von Rillen auf der dem Unterteil (2) bzw. dem Oberteil (3) gegenüberliegenden Seite ausgeformt ist.

2. Zweiteiliges Kunststoffrohling-Set nach Anspruch 1, wobei die Aufnahmeraumausformung (12) kugelkappenförmig oder kugelabschnittförmig ist.

3. Zweiteiliges Kunststoffrohling-Set nach einem der vorangehenden Ansprüche, wobei das Deckelteil (3) im wesentlichen eben ausgebildet ist.

4. Wirkstoffapplikator, der durch Verschweißen, vorzugsweise Ultraschall-Verschweißen, des Unterteils (2) mit dem Deckelteil (3) des zweiteiligen Kunststoffrohling-Sets nach einem der Ansprüche 1 bis 3 über die jeweiligen Siegelränder (5, 6) herstellbar ist und mit einem Wirkstoff befüllt ist.

## Claims

1. Two-part plastic blank set, preferably for producing an active substance applicator (1) for storing a unit dose of an active substance preparation and for administering this unit dose into the human nose, wherein the plastic blank set comprises a base part (2) and lid part (3) that is separate from the base part (2), wherein the base part (2) has a receiving space moulding (12) encompassed by a sealing rim (5) and an application tube (7) moulded on integrally extends from the receiving space moulding (12) and is closed at its free end with a closure (10) that is moulded on integrally via an attachment element (15), wherein the attachment element (15) has a wall thickness of 0.1-0.5 mm, and wherein the lid part (3) is of a size such that when placed onto the base part (2) it completely covers the receiving space moulding (12) thereof, and has a sealing rim (6) that can be connected to the sealing rim (5) of the base part (2) to form a seal,
wherein an attachment tab (16) extending from the respective sealing rim (5, 6) is moulded on at least at the lid part (3) or at the base part (2), and
wherein at least the sealing rim (6) of the lid part (3) or the sealing rim (5) of the base part (2) is moulded with an energy director (17) in the form of grooves on the side opposite the base part (2) or the top part (3).

2. Two-part plastic blank set according to claim 1, wherein the receiving space moulding (12) is in the shape of a spherical cap or in the shape of a spherical segment.

3. Two-part plastic blank set according to one of the preceding claims, wherein the lid part (3) is essentially of a flat design.

4. Active substance applicator which can be produced through welding, preferably ultrasonic welding, of the base part (2) to the lid part (3) of the two-part plastic blank set according to one of the claims 1 to 3 via the respective sealing rims (5, 6) and is filled with an active substance.

## Revendications

1. Une ébauche en matière plastique et en deux parties, de préférence pour produire un applicateur de substance active (1) afin de stocker une dose unitaire d'une préparation à base de substance active et fin d'administrer cette dose unitaire dans une narine humaine et cette ébauche en matière plastique se compose d'un socle (2) et d'un couvercle (3) qui est séparé du socle (2), et ce socle (2) comporte un espace moulé de réception (12) qui est entouré d'un rebord d'étanchéité (5) et a un tube d'application (7) qui est intégré par moulage et se prolonge depuis l'espace moulé de réception (12) et est clos, au niveau de son extrémité libre par une fermeture (10) qui est intégrée par moulage par le biais d'un élément d'attachement (15), et cet élément d'attachement (15) a une épaisseur de paroi comprise entre 0,1 et 0,5 mm, et le couvercle (3) a une taille qui lui permet, lorsqu'il est placé sur le socle (2), de recouvrir complètement son espace moulé de réception (12), et a un rebord d'étanchéité (6) qui peut se rattacher au rebord d'étanchéité (5) du socle (2) afin de former un joint,
et une languette de rattachement (16), qui part du rebord d'étanchéité respectif (5, 6) vient se fixer par moulage, à titre minimum, au couvercle (3) ou au socle (2) et,
à titre minimum, le rebord d'étanchéité (6) du couvercle (3) ou le rebord d'étanchéité (5) du socle (2) comporte un directeur moulé d'énergie (17) qui se présente sous la forme de rainures implantées sur le côté qui fait face au socle (2) ou à la partie la plus haute (3).

2. L'ébauche en matière plastique et en deux parties que décrit la revendication 1, si ce n'est que l'espace moulé de réception (12) se présente sous la forme d'un chapeau sphérique ou sous la forme d'un segment sphérique.

3. L'ébauche en matière plastique et en deux parties que décrit l'une ou l'autre des revendications précédentes, si ce n'est que le couvercle (3) a un profil essentiellement plat.

4. Un applicateur de substance active qui peut être produit par soudage, de préférence par soudage ultrasonique du socle (2) sur le couvercle (3) de l'ébauche en matière plastique et en deux parties que décrit l'une ou l'autre des revendications 1 à 3 via les rebords d'étanchéité (5, 6) respectifs et est rempli d'une substance active.
